# EUROPEAN PATENT APPLICATION

(11) **EP 4 306 508 A1**
(43) Date of publication of application: **17.01.2024**
(21) Application number: 22184885.6
(22) Date of filing: 14.07.2022
(51) Int. Cl.: C07C 231/24, C07C 233/03

(54) **IMPROVED PROCESS FOR RECOVERING N,N-DIMETHYLFORMAMIDE (DMF) FROM AN AQUEOUS PROCESS STREAM**

(71) Applicant: BASF Agro B.V., 6811 AH Arnhem (NL)
(72) Inventor: Wang, Chao, McIntosh, Alabama 36553-0113 (US); Cortes, David A., Quincy, 62305 (US); O'Hara, Sean P., Palmyra, Missouri 63461 (US); Kelleher, Patrick, Houston, Texas 77079 (US); Scheele, Erika, Houston, Texas 77079 (US); Ribeiro, Claudio P., Houston, Texas 77079 (US)
(74) Representative: BASF IP Association

(57) **Abstract**

The invention relates to an improved process for recovering *N*,*N*-Dimethylformamide (DMF) from an aqueous process stream by extracting with substituted phenols and subsequent distillation.

## Description

The present invention relates to a new and highly efficient process for recovering *N*,*N-*Dimethylformamide (DMF) from aqueous process streams.

*N*,*N*-Dimethylformamide (DMF) ((CH₃)₂NC(O)H) is a polar aprotic solvent with a high boiling point (153°C), a low evaporation rate (low volatility), and high miscibility with water. As a comparably cheap solvent that promotes numerous reactions, DMF has been employed extensively for decades in a variety of chemical processes, ranging from the industrial production of acrylic fibers and plastics to the development and production of biologically active compounds such as pharmaceuticals and pesticides.

In parallel, processes for recovering DMF from process or waste streams, e.g. by extraction and/or distillation/rectification or pervaporation, have been developed to save raw materials, to reduce cost and to minimize environmental impact:
GB1589793 discloses a process for isolating a lower alkylamide such as DMF by extraction from its aqueous solution with an extractant and subsequent distillation of the resulting mixture. According to GB1589793, suitable extractants are substituted phenols of the general formula II 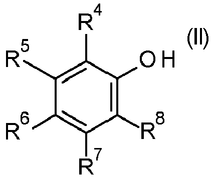 where R⁴ to R⁸ are identical or different and each is hydrogen, alkyl, cyclopentyl or cyclohexyl, with the proviso that the sum of the carbon atoms of R⁴ to R^{s} is from 3 to 9. All phenols according to GB1589793 used as extractant have a boiling point at atmospheric pressure differing from that of DMF by 70°C to 130°C.

According to Rush, F. et al. methylene chloride is superior to many other solvents examined for isolating lower alkyl amides from aqueous solutions, including carbon tetrachloride, chloroform, trichloroethylene, ethyl acetate, isopropyl ether, butyl acetate, benzene and heptane, Rush, F. E., Olson, J. H., In Smith, B. D., E.I. du Pont de Nemours & Company., & University of Delaware. (1972). DMF recovery and purification: Preliminary design and economic evaluation. Wilmington Del..

CN1317259 discloses a process for recovering dimethylformamide in wastewater by extraction and rectification, using an organic solvent with a boiling point difference to DMF, which is more than or equal to 80°C, e.g. carbon tetrachloride, 1,1 dichloroethane, 1,1,1 trichloroethane, 1,2 dichloroethylene, chloropropane, or trichloropropene.

Chlorinated hydrocarbons like dichloromethane, chloroform, dichloroethane, or carbon tetrachloride were suggested as extractants for DMF from aqueous waste stream in CN101397260, CN106397252, CN108862795, CN108059291, and CN111470997.

In addition to chlorinated hydrocarbons, Dou et al. tested benzene, toluene and long chain alcohols like 1-octanol, 1-nonanol, and 1-decanol as extractants for the removal of DMF from wastewater (Process Safety and Environmental Protection 130 (2019) 317-325).

CN111100028 teaches an extraction-rectification method for DMF using octanoic acid, eugenol, phenol, o-sec-butylphenol or nonylphenol as extractants.

Xiaoyu Wang et al. examined how the presence of salts such as NaCl or Na₂SO₄ affects extraction of DMF with chloroform (J. Chem. Eng. Data 2021, 66, 2233-2243).

The use of chlorinated extractants for recovering DMF from aqueous solutions has several disadvantages, e.g. the formation of small amounts of hydrogen chloride during the workup of both the extract phase and the raffinate by distillation. Therefore, the equipment must be constructed from highly corrosive resistant, expensive materials. In addition, the low distribution coefficient of DMF in the chlorinated solvents of about 0.3-0.5 requires large volumes of solvent and a large number of extraction steps to isolate DMF efficiently. Extraction is recommended for recovering DMF in concentrations below 10% in the aqueous solution.

Numerous publications teach different solvents to recover DMF from aqueous systems, such as industrial wastewater, however, the results still do not meet today's requirements for economic, safe and practical processes.

DMF is a suitable solvent in the process for the production of 3-Phenyluracils, which are important intermediates in the process for the preparation of herbicidally active compounds such as Saflufenacil, an inhibitor of the plant enzyme Protoporphyrinogen Oxidase (PPO).

WO2006/010474 inter alia discloses a process for the preparation of a compound of formula (I), a 3-Phenyluracil, as shown in Figure 1 below.

### Figure 1:

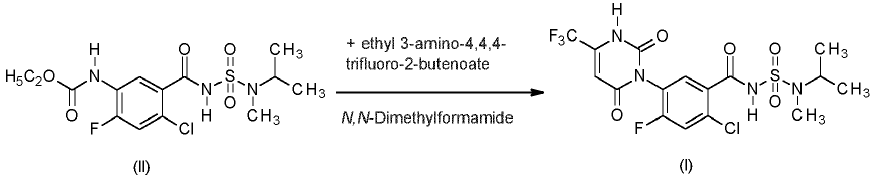

*N*,*N*-Dimethylformamide is a suitable solvent in this process. After completion of the reaction, the reaction mixture is added dropwise with cooling to a dilute inorganic acid, such as hydrochloric acid or sulfuric acid. The compound of formula (I) precipitates, is filtered off, washed and dried. The remaining mother liquor (ML) comprises the solvent *N,N-*Dimethylformamide, water, salts and organic compounds related to the reaction, including byproducts.

The mother liquor is a waste product, whose adequate disposal is troublesome and costly. For economic and environmental reasons, it is therefore highly desirable to efficiently recover *N,N-*Dimethylformamide from the mother liquor for reuse in the process according to Figure 1 or any other chemical process, as appropriate.

Accordingly, it is an object of the present invention to provide a highly efficient process for recovering the solvent *N*,*N*-Dimethylformamide in high yield and with high purity from aqueous process or waste streams, which comprise inorganic salts and/or organic compounds. In particular, it is an object of the present invention to provide a highly efficient process for recovering the solvent *N*,*N*-Dimethylformamide in high yield and with high purity from aqueous process or waste streams, which comprise inorganic salts and/or high molecular weight organic compounds related to preceding reactions, including byproducts.

Furthermore, it is an object of the present invention to provide a highly efficient process for the production of 3-Phenyluracil intermediates, wherein the solvent *N*,*N*-Dimethylformamide is recovered in high yield and with high purity from the aqueous mother liquor (ML) after precipitation of the 3-Phenyluracil intermediate. In particular, it is an object of the present invention to provide a highly efficient process for the production of the compound of formula (I) 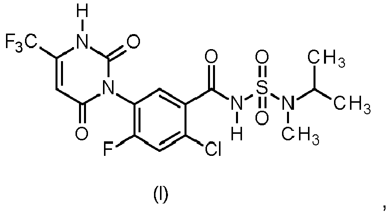 wherein the solvent *N*,*N*-Dimethylformamide is recovered in high yield and with high purity from the aqueous mother liquor (ML) after precipitation of the compound of formula (I). It is a further object of the invention to recover the solvent *N*,*N*-Dimethylformamide with such high purity from the aqueous mother liquor, that it can be reused in the process for the production of the compound of formula (I). In particular, it is an object of the invention to recover the solvent *N,N-*Dimethylformamide from aqueous solutions substantially free of unwanted impurities such as salts and/or high molecular weight organic compounds related to the reaction, including byproducts.
These and further objectives are achieved by the process described below.

DMF is frequently used as a polar aprotic solvent to promote desired reactions in pesticide manufacturing. Often, the work up of the reaction consists of adding water to precipitate a solid or to extract the product with a water immiscible organic solvent. DMF mostly remains in the aqueous phase and is difficult to recover by distillation since water is lower boiling than DMF and there is usually the presence of a high level of inorganic salts that prevents a DMF recovery of even 50% before salt crystallization occurs. DMF is therefore typically incinerated which contributes to unwanted emissions. Ideally, an organic solvent would extract out all DMF from the aqueous waste stream, leaving the salts and other unwanted impurities such as salts and/or high molecular weight organic compounds related to the reaction, including byproducts, in the aqueous stream with a minimal amount of water in the extracted DMF. The extractant could then be distilled from DMF and recycled, if it has a lower boiling point than DMF, or DMF can be distilled away from a high-boiling solvent. If necessary, DMF could be dried for reuse.

Accordingly, the present invention provides a process for recovering *N*,*N*-Dimethylformamide ((CH₃)₂NC(O)H) from aqueous solutions and separating *N*,*N*-Dimethylformamide from unwanted impurities in the aqueous solution, comprising the following steps A) and B):

### Process steps A) and B):

In a first step A), DMF is extracted from an aqueous process stream with an extractant selected from substituted phenols of the general formula (Ph0), 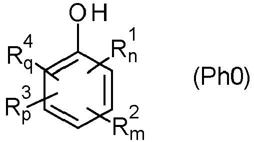 wherein
R¹ is methyl;
R² is ethyl;
R³ is halogen selected from F, Cl, Br, and I;
R⁴ is nitro;
n is 0, 1, or 2;
m is 0, or 1;
p is 0, 1, 2, or 3;
q is 0, 1, or 2; and
wherein at least one of n, m, p or q is not 0; and
wherein m is 0, if n is 1 or 2; and
wherein n is 0, if m is 1.

Preferred extractants are substituted phenols of formula (Ph1): 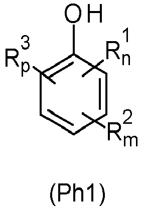 wherein
R¹ is methyl;
R² is ethyl;
R³ is halogen selected from F, Cl, Br, and I, preferably Cl;
n is 0, 1, or 2;
m is 0, or 1;
p is 0, 1, 2, or 3;
wherein at least one of n, m, or p is not 0; and
wherein m is 0, if n is 1 or 2; and
wherein n is 0, if m is 1.

Particularly preferred extractants are substituted phenols of formulae (Ph1.1) to (Ph1.3)

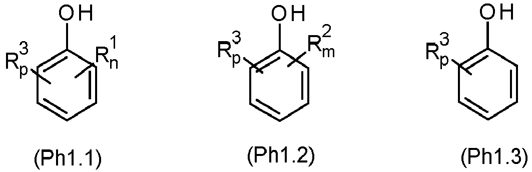

In the phenols of formula (Ph1.1),
R¹ is methyl;
R³ is halogen selected from F, Cl, Br, and I, preferably Cl;
n is 1, or 2;
p is 0, 1, 2, or 3, preferably 0;
In the phenols of formula (Ph1.2),
R² is ethyl;
R³ is halogen selected from F, Cl, Br, and I, preferably Cl;
m is 1;
p is 0, 1, 2, or 3, preferably 0;
In the phenols of formula (Ph1.3),
R³ is halogen selected from F, Cl, Br, and I, preferably Cl;
p is 1, 2, or 3, preferably 1;

More preferred extractants are substituted phenols of formulae (Ph1.1.a)-(Ph1.1.d), (Ph1.2.a)-(Ph1.2.c), and (Ph1.3.a)-(Ph1.3.c):

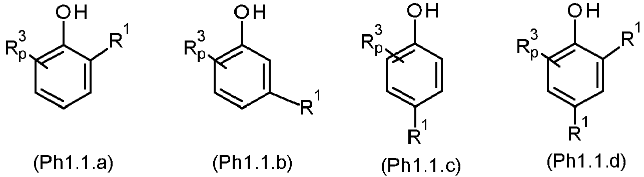

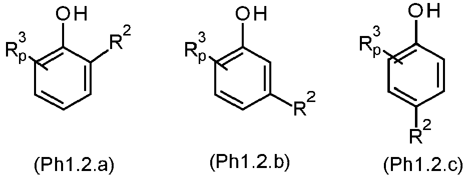

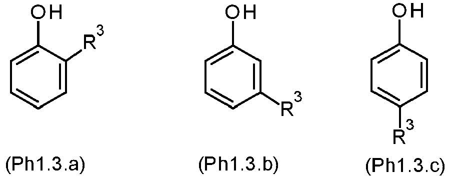

In the phenols of formula (Ph1.1.a)-(Ph1.1.d),
R¹ is methyl;
R³ is halogen selected from F, Cl, Br, and I, preferably Cl;
p is 0, 1, 2, or 3, preferably 0;
In the phenols of formula (Ph1.2.a)-(Ph1.2.c),
R² is ethyl;
R³ is halogen selected from F, Cl, Br, and I, preferably Cl;
p is 0, 1, 2, or 3, preferably 0;
In the phenols of formula (Ph1.3.a)-(Ph1.3.c),
R³ is halogen selected from F, Cl, Br, and I, preferably Cl;

Individual phenols which are most suitable as extractant in the process according to the invention are
- 3-methylphenol (meta-cresol, m-cresol): boiling point: 202.8°C; melting point: 11-12°C 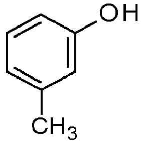
- 3-ethylphenol: boiling point: 218.4°C; melting point: - 4.5°C 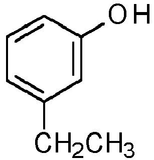
- 2-chlorophenol: boiling point: 174,9 °C; melting point: 9,4°C

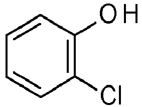

The substituted phenols used are either known or readily obtainable by conventional methods. If their solidification point is above the extraction temperature, either a lower-melting mixture of substituted phenols or an additional solvent, preferably a hydrocarbon boiling at up to120°C at atmospheric pressure, e.g. n-hexane and its isomers, cyclohexane, methylcyclohexane or n-heptane and its isomers, is used.

The water content in the aqueous process stream is highly variable, depending on the respective process. Normally, the water content of the aqueous process stream is more than 30% [w/w]. Preferably, the water content of the aqueous process stream is more than 50% [w/w] or 60% [w/w], in particular more than 70% [w/w]. The pH of the aqueous process stream or the wastewater is widely variable. The extraction of DMF can be performed with high efficiency from an aqueous process stream or the wastewater with acidic, neutral or basic pH value. If appropriate, the pH can be adjusted to a neutral or basic value by adding a suitable inorganic base, e.g. KOH, NaOH, etc., preferably 50% caustic soda, and to a neutral or acidic value by adding a suitable acid, e.g. sulfuric acid, hydrochloric acid or acidic acid etc.. In one embodiment, the pH is adjusted to equal to or higher than 2. In one embodiment, the pH is adjusted to equal to or higher than 7. In another embodiment, the pH is adjusted to equal to or higher than 8. In another embodiment, the pH is adjusted to equal to or higher than 9. In another embodiment, the pH is adjusted to equal to or higher than 10, e.g. to 11.

The concentration of DMF in the aqueous process stream is between 1-30% [w/w], preferably between 1-20% [w/w], and most preferably between 5-20% [w/w]. Depending on the respective process, the aqueous process stream may comprise impurities, e.g. inorganic salts or organic compounds related to the reaction. An example for inorganic salts is sodium sulfate (Na₂SO₄). If present, the concentration of the inorganic salts may be up to 15% [w/w], e.g. between 5 and 15% [w/w] or 7 and 10% [w/w]. The aqueous process stream may comprise organic compounds, e.g. alcohols and other organic solvents, generally in an amount of less than 5% [w/w]. The aqueous process stream may comprise high molecular weight organic compounds related to the reaction, including byproducts.

The extraction may be carried out batchwise or continuously, in accordance with conventional techniques, and at temperatures from 10 to 80°C, preferably from 20 to 60°C, more preferably from 20 to 40°C. In continuous operation, counter-current extraction is preferred. The weight ratio of the extractant to the aqueous process stream is from 0.1:1 to 2:1, preferably from 0.25:1 to 2:1, more preferably from 0.25:1 to 1.5:1, and in particular from 0.25:1 to 1:1. The absolute volumes are variable and depend on the volume of the reaction batch and the equipment. After phase separation, the organic phase is removed. Optionally, the aqueous phase can be extracted with a fresh amount of the extractant. The number of washes depends on the weight ratio of extractant and aqueous phase and the desired recovery rate for DMF. Generally, the number of extractions or washes is between 1 and 5, preferably between 1 and 3, more preferably 1 or 2.
If appropriate, the organic phases are combined for subsequent distillation or rectification.

In a second step B), the components of the organic phase from step A) are separated by distillation/rectification in accordance with conventional techniques, either batchwise or continuously. Distillation takes place at atmospheric pressure or under vacuum, preferably under vacuum (e.g. 1 - 30 kPa, preferably 3 - 15 kPa), and temperatures between 100 - 220°C (top - bottom of tower; preferably below 150°C).

Figure 2 shows a flow diagram of this process.

The organic extract contains almost no salts or water. The extractant is recovered by distillation for reuse. The extractant distillation also removes any entrained water and lower boiling components like alcohols such that the recovered DMF can be used directly in a subsequent reaction, e.g. in the process for the preparation of the compound of formula (I).

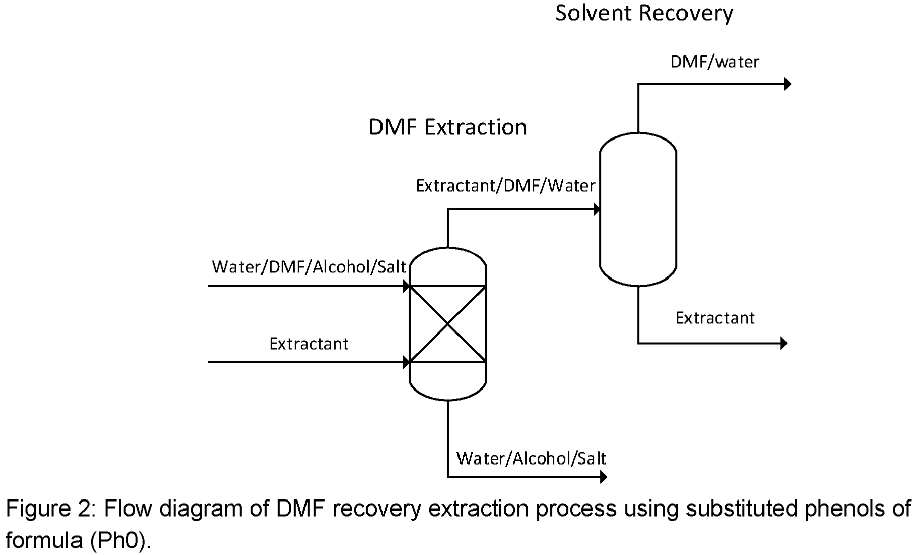

The process of the present invention is also useful in a process for the production of 3-Phenyluracils according to Figure 1 above.

Accordingly, the following embodiments are embodiments of the invention:
i) A process for the production of 3-Phenyluracils, wherein the solvent *N*,*N-*Dimethylformamide (DMF) is recovered from an aqueous process stream after precipitation and filtration of the 3-Phenyluracils comprising the steps of
   A). Extracting the aqueous process stream with substituted phenols of the general formula (Ph0), 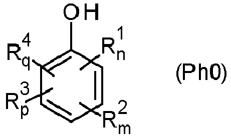 wherein
      R¹ is methyl;
      R² is ethyl;
      R³ is halogen selected from F, Cl, Br, and I;
      R⁴ is nitro;
      n is 0, 1, or 2;
      m is 0, or 1;
      p is 0, 1, 2, or 3;
      q is 0, 1, or 2; and
      wherein at least one of n, m, p or q is not 0; and
      wherein m is 0, if n is 1 or 2; and
      wherein n is 0, if m is 1; and
   B). Recovering DMF from the organic phase by distillation and/or rectification,
ii) The process as claimed in embodiment i), wherein the substituted phenol is m-cresol.
iii) The process as claimed in embodiment i), wherein the substituted phenol is 2-chlorophenol.
iv) The process as claimed in any of embodiments i)-iii), wherein the ratio of the substituted phenol to the aqueous process stream is from 0.1:1 to 2:1.
v) The process as claimed in any of embodiments i)-iii), wherein the ratio of the substituted phenol to the aqueous process stream is from 0.5:1 to 1,5:1.
vi) The process as claimed in any of embodiments i)-v), wherein the extraction is carried out at a temperature between 20 to 80°C.
vii) The process as claimed in any of embodiments i)-v), wherein the extraction is carried out at a temperature between 20 to 60°C.
viii) The process as claimed in any of embodiments i)-vii), wherein the number of extractions is 1 to 2.
ix) The process as claimed in any of embodiments i)-viii), wherein the distillation and/or rectification is carried out under atmospheric pressure.
x) The process as claimed in any of embodiments i)-viii), wherein the distillation and/or rectification is carried out under vacuum.
xi) The process as claimed in any of embodiments i)-x), wherein the aqueous process stream comprises high molecular weight organic impurities, including byproducts.
xii) The process as claimed in any of embodiments i)-xii), wherein the recovered DMF is reused in the process for the production of 3-Phenyluracils.

### Examples:

### Example 1

M-cresol has been identified as a suitable solvent to recover DMF from wastewater streams containing inorganic salts in a single stage extraction system. The m-cresol to wastewater mass ratio used in the liquid-liquid extraction was 1:1. Aqueous dimethylformamide (DMF) solution (400 g) containing 11.8% [w/w] of DMF was extracted with 400 g of m-cresol in a 1.0 L extraction cell at pH 11 and 40°C (total volume about 800 ml). The DMF solution to be extracted (ML) originated from a pesticide production waste stream. After mixing for 3-5 mins with agitation, the mixture was allowed to settle. Upon phase split, 514 g of organic extract phase containing 7.2% [w/w] of DMF, 10.7% [w/w] of water, 1.4% [w/w] methanol, 0.7% [w/w] ethanol, and 279 g of raffinate containing 81.3% [w/w] of water, 0.0%[w/w] of DMF, 1.4% [w/w] methanol and 0.0% [w/w] ethanol were obtained. The recovery yield of DMF was 100% in a one-stage extraction (see Ex.1.1 in Table 1 below).

Ex. 1.2 - 1.7: In analogy to Example 1 described above, further experiments were conducted in accordance with Table 1 below:
- # of washes: 1
- Extractant: 3-methylphenol (m-cresol)

**Table 1:**

| No. | Extractant: aqu. sol. | T [°C] | pH | DMF% [w/w] |
|---|---|---|---|---|
| 1.1 | 1:1 | 40 | 11 | 100 |
| 1.2 | 0.75:1 | 40 | 11 | 96,3 |
| 1.3 | 0.5:1 | 40 | 11 | 92,1 |
| 1.4 | 0.25:1 | 40 | 11 | 67,9 |
| 1.5 | 0.5:1 | 40 | 2 | 85,4 |
| 1.6 | 0.5:1 | 40 | 7 | 90,6 |
| 1.7 | 0.5:1 | 60 | 7 | 90,4 |

3-methylphenol (m-cresol) was found to extract virtually all the DMF with a single stage extraction of equal weight. In this case, the relatively small amount of water in the extract can be removed by distillation before the DMF can be distilled off from the higher boiling 3-methylphenol, while leaving undesired impurities in the bottoms. Some 3-methylphenol remains in the aqueous phase, but this can be recovered by an azeotropic distillation.

### Example 2

2-chlorophenol has been identified as a suitable solvent to recover DMF from wastewater streams containing inorganic salts in a single stage extraction system. The 2-chlorophenol to wastewater mass ratio used in the liquid-liquid extraction was 1:1. Aqueous dimethylformamide (DMF) solution (80.0 g) containing 14.2% [w/w] of DMF was extracted with 80.0 g of 2-chlorophenol in a 0.5 L extraction cell at pH 10.7 and 40°C (total volume about 160 ml). The DMF solution to be extracted (ML) originated from a pesticide production waste stream. After mixing for 3-5 mins with agitation, the mixture was allowed to settle. Upon phase split, 101.2 g of organic extract phase containing 9.9% [w/w] of DMF, 9.6% [w/w] of water, 1.5% [w/w] methanol, 0.6% [w/w] ethanol, and 56.0 g of raffinate containing 82.5% [w/w] of water, 0.0%[w/w] of DMF, 2.3% [w/w] methanol and 0.0% [w/w] ethanol were obtained. The recovery yield of DMF was 100% in a one-stage extraction.

## Claims

1. A process for recovering *N*,*N*-Dimethylformamide (DMF) from an aqueous process stream comprising the steps of
A. Extracting the aqueous process stream with substituted phenols of the general formula (Ph0), 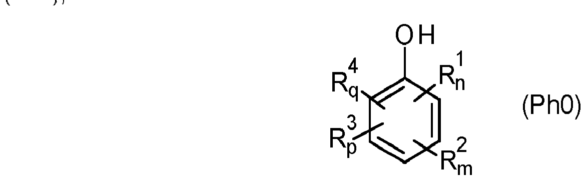 wherein
R¹ is methyl;
R² is ethyl;
R³ is halogen selected from F, Cl, Br, and I;
R⁴ is nitro;
n is 0, 1, or 2;
m is 0, or 1;
p is 0, 1, 2, or 3;
q is 0, 1, or 2; and
wherein at least one of n, m, p or q is not 0; and
wherein m is 0, if n is 1 or 2; and
wherein n is 0, if m is 1; and
B. Recovering DMF from the organic phase by distillation and/or rectification,

2. The process as claimed in claim 1, wherein the ratio of the substituted phenol to the aqueous process stream is from 0.1:1 to 2:1.

3. The process as claimed in claim 1, wherein the ratio of the substituted phenol to the aqueous process stream is from 0.5:1 to 1,5:1.

4. The process as claimed in anyone of the preceding claims, wherein the extraction is carried out at a temperature between 20 to 80°C.

5. The process as claimed in anyone of the preceding claims, wherein the number of extractions is 1 or 2.

6. The process as claimed in anyone of claims 1 to 5, wherein the substituted phenol is m-cresol.

7. The process as claimed in anyone of claims 1 to 5, wherein the substituted phenol is 2-chlorophenol.

8. The process as claimed in anyone of the preceding claims, wherein the distillation and/or rectification is carried out under atmospheric pressure.

9. The process as claimed in anyone of the preceding claims, wherein the distillation and/or rectification is carried out under vacuum.

10. The process as claimed in anyone of the preceding claims, wherein the distillation and/or rectification is carried in a continuous process.

11. The process as claimed in anyone of the preceding claims, wherein the aqueous process stream comprises high molecular weight organic impurities, including byproducts.
